# EUROPEAN PATENT SPECIFICATION

(11) **EP 3 053 507 B1**
(45) Date of publication and mention of the grant of the patent: **18.10.2017**
(21) Application number: 15183043.7
(22) Date of filing: 28.08.2015
(51) Int. Cl.: A61B 1/00

(54) **MEDICAL PROBE**
MEDIZINISCHE SONDE
SONDE MÉDICALE

(30) Priority: 04.02.2015 TW 104103805
(43) Date of publication of application: 10.08.2016
(73) Proprietor: EDA Medical Devices Technology Inc., Taichung City 428 (TW)
(72) Inventor: CHIANG, Tai-An, 428 Taichung City (TW); UEN, Tzeng-Ming, 428 Taichung City (TW)
(74) Representative: HGF Limited

(56) References cited:
- US-A- 3 556 085
- US-A- 4 269 192
- US-A- 4 566 438
- US-A- 5 385 572
- US-A1- 2003 144 594

## Description

The disclosure relates to a medical probe, and more particularly to a medical probe adapted for examining a body cavity of a patient during surgery.

A conventional medical probe device for penetrating and examining a body cavity of a patient is generally applied to a minimally invasive surgery (e.g., a front artificial pneumoperitoneum) in order to improve accuracy of a penetrating process during the surgery. Referring to Figure 1, the medical probe device 1, as disclosed in Taiwanese Patent No. 250437, includes a probe body 11 and an optical fiber 12. The probe body 11 has an end part 111 that is formed at a front end of the probe body 11 and a concave part 112 that is formed at one side of the probe body 11. The optical fiber 12 has a viewing tip 121 at a front end of the optical fiber 12. The viewing tip 121 of the optical fiber 12 is may be disposed at the end part 111, or in the concave part 112 of the probe body 11 such that the viewing tip 121 may capture images in front of the probe body 11 during the penetrating process of the surgery.

However, if the viewing tip 121 is disposed in the concave part 112 of the probe body 11, because the viewing angle of the viewing tip 121 may be limited due to blockage by the end part 111 of the probe body 11, it will be difficult to verify an image incident on the viewing tip 121.

If the viewing tip 121 is disposed at the end part 111 of the probe body 11 in order to obtain a wider viewing angle, the end part 111 requires an additional part for supporting the viewing tip 121, which can increase a cross-section of the end part 111 and thus reduce a penetrating effect of the probe body 11.

US 3 556 085 A discloses a microscope adapted to be inserted into the spaces in a living body for direct examination of the interior surfaces thereof and having a magnifying objective lens system and a fiber optical viewing system.

US 4 566 438 A discloses a fiber-optic stylet for use with a surgical needle. The stylet includes a transmitting light fiber having a beveled end.

US 5 385 572 A discloses a trocar having a pathway for light communication from a cutting element of the trocar through a shaft of the trocar.

US 2003/144594 A1 discloses a needle device having an optical element axially positioned in a nozzle of the needle device for transmitting and receiving optical radiation through the nozzle.

Therefore, an object of the disclosure is to provide a medical probe that can alleviate at least one of the drawbacks of the prior arts.

In accordance with the present invention, there is provided a medical probe for examining a body cavity during surgery, as claimed in claim 1.

According to the disclosure, a medical probe is for examining a body cavity during surgery. The medical probe includes a hollow needle body, a light-transmitting optical module, an imaging module and a microelectro-mechanical module. The hollow needle body defines a needle passage that extends along an axis of the hollow needle body, and includes an outer tubular wall that surrounds the needle passage. The outer tubular wall includes a penetrating section that is formed with a forward penetrating end and that has a cross-section smaller than a remainder of the outer tubular wall, and a needle opening that is formed in the penetrating section and that is communicated with the needle passage.

The light-transmitting optical module is disposedinside the needle passage, and includes a light entering region that extends to and exposed from the needle opening to receive light from the body cavity. The light-transmitting optical module transmits the light within the needle passage.

The imaging module is disposed in the needle passage to capture an image carried by the light transmitted through the light-transmitting optical module.

Other features and advantages of the disclosure will become apparent in the following detailed description of the embodiments with reference to the accompanying drawings, of which:

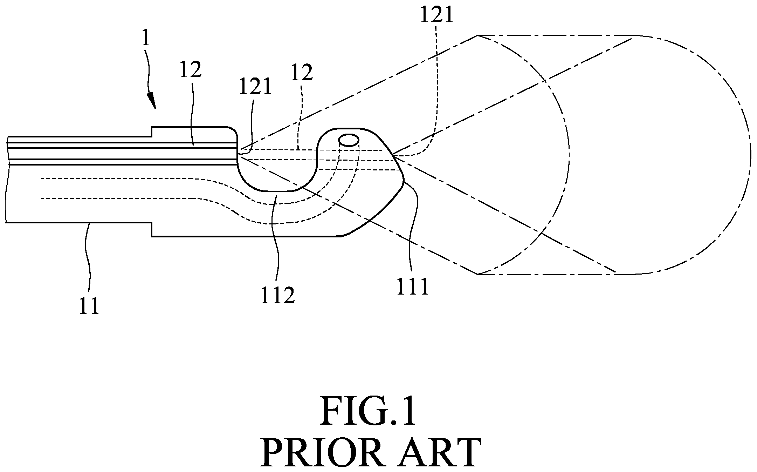

Figure 1 is a fragmentary cross-sectional view of a medical probe device disclosed in Taiwanese Patent No. 250437.
Figure 2 is a fragmentary exploded perspective view of a first embodiment of a medical probe according to the present disclosure;
Figure 3 is a fragmentary perspective view of the first embodiment according to the present disclosure;
Figure 4 is a fragmentary sectional view of the first embodiment according to the present disclosure;
Figure 5 is a cross-sectional view of the medical probe taken along line V-V in Figure 4;
Figure 6 is a fragmentary schematic view of a second embodiment of the medical probe according to the present disclosure; and
Figure 7 is a fragmentary sectional view of a third embodiment of the medical probe according to the present disclosure.

Before the disclosure is described in greater detail, it should be noted that like elements are denoted by the same reference numerals throughout the disclosure.

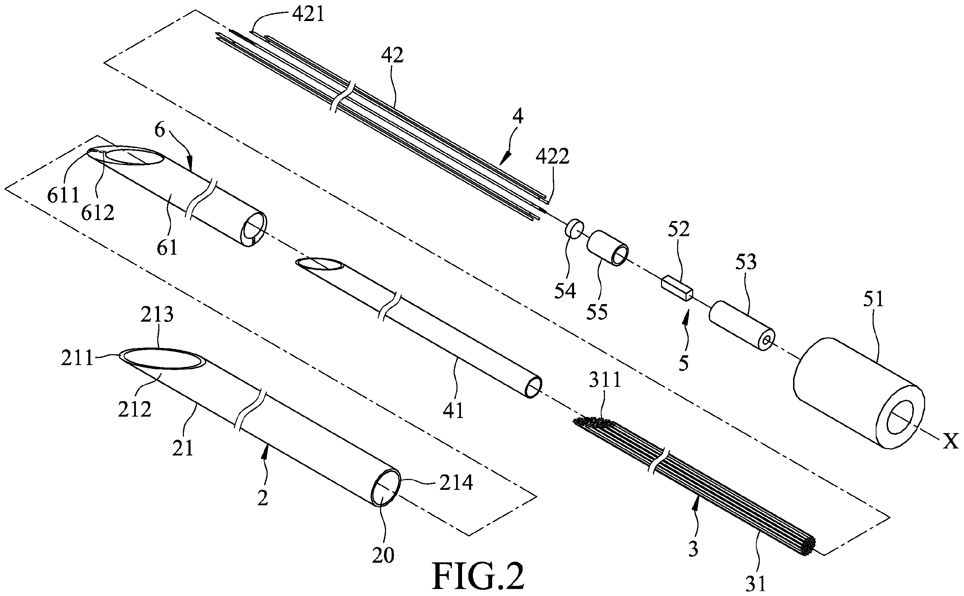

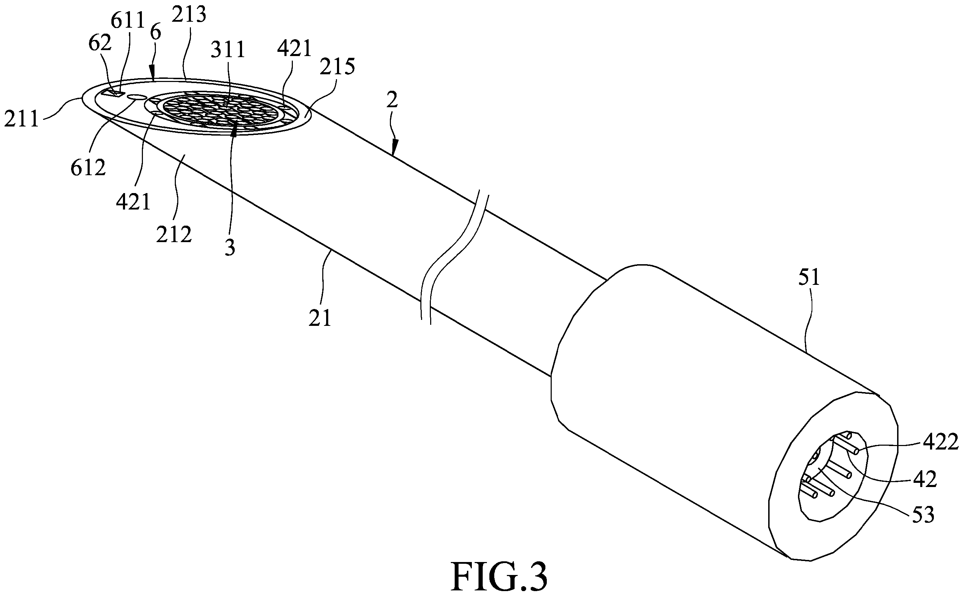

Referring to Figures 2 to 5, a first embodiment of a medical probe according to the present disclosure is used for examining a body cavity of a patient. The medical probe includes a hollow needle body 2, a light-transmitting optical module 3, a light-emitting module 4, an imaging module 5 and a microelectro-mechanical module 6.

The hollow needle body 2 defines a needle passage 20 extending along an axis (X) of the hollow needle body 2, and includes an outer tubular wall 21 surrounding the needle passage 20. The outer tubular wall 21 includes a penetrating section 212 that is formed with a forward penetrating end 211 and that has a cross-section smaller than a remainder of the outer tubular wall 21, and a rearward end 214 opposite to the forward penetrating end 211. The outer tubular wall 21 is formed, in the penetrating section 212 thereof, with a needle opening 213 that is communicated with the needle passage 20. In this embodiment, the outer tubular wall 21 is beveled to form the penetrating section 212 so that the penetrating section 212 has a beveled annular surface 215 that is inclined with respect to the axis (X) of the hollow needle body 2 and that confines the needle opening 213. The forward penetrating end 211 of the penetrating section 212 of the outer tubular wall 21 is situated at one side of the beveled annular surface 215 offset from the axis (X) of the hollow needle body 2. The hollow needle body 2 further includes an adapter 51 that is connected to the rearward end 214 of the outer tubular wall 21. The needle passage 20 of the needle body 2 is surrounded by the outer tubular wall 21 and the adapter 51.

The light-transmitting optical module 3 is disposed inside the needle passage 20 of the hollow needle body 2, and includes a plurality of optical fibers 31. The optical fibers 31 are disposed inside the needle passage 20 of the hollow needle body 2 along the axis (X) between the forward penetrating end 211 and the imaging module 5. The optical fibers 31 are beveled to respectively have beveled surfaces that cooperatively form a light entering region 311 in the needle opening 213 to receive light from the body cavity. The light-transmitting optical module 3 transmits the light within the needle passage 20.

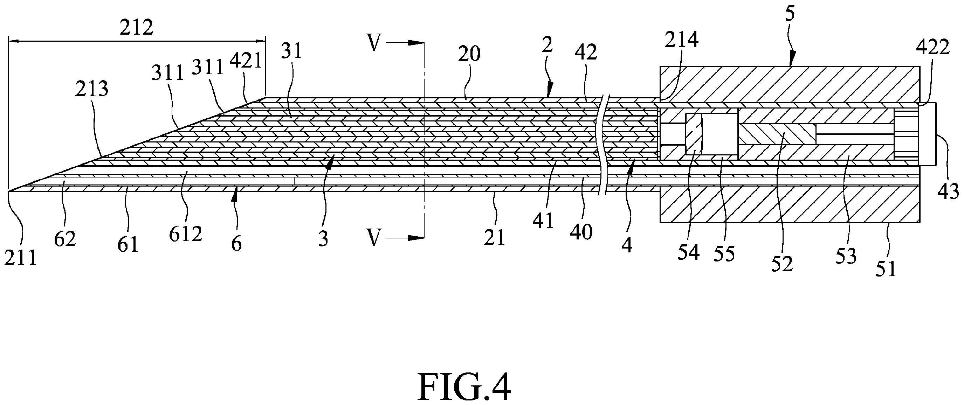

The light-emitting module 4 is to emit light to pass through the needle passage 20 and to deliver the light to the body cavity through the needle opening 213. The hollow needle body 2 further includes an inner tubular wall 41 that is disposed within the outer tubular wall 21 along the axis (X) of the hollow needle body 2 and that surrounds the optical fibers 31 of the light-transmitting optical module 3. The light-emitting module 4 includes a plurality of light guide rods 42 that extend along the axis (X) of the hollow needle body 2 and that are disposed around the inner tubular wall 41, and a light emitter 43 that is distal from the needle opening 213 (particularly, at a rearward end of the adapter 51 as shown in Figure 4). Each of the light guide rods 42 has a light entrance end 422 that is proximal to the light emitter 43 to receive light emitted from the light emitter 43, and a light exit end 421 that extends to and is exposed from the needle opening 213 of the hollow needle body 2 to deliver the light through the needle opening 213 to the body cavity. The inner tubular wall 41 cooperates with the outer tubular wall 21 to define a passageway 40 that extends along the axis (X) of the hollow needle body 2. The light emitter 43 may be one of a light-emitting diode (LED), laser, cool light, etc.

The imaging module 5 is disposed in the needle passage 20 (particularly, in a portion of the needle passage 20 surrounded by the adapter 51) to capture an image carried by the light transmitted through the light-transmitting optical module 3. The imaging module 5 includes an image sensor 52 that is disposed inside the adapter 51, a sensor carrier 53 that is inserted into the adapter 51 and that carries the image sensor 52, a lens 54 that is disposed between the light-transmitting optical module 3 and the image sensor 52 to focus the light, and a lens carrier 55 that is inserted into the adapter 51 and that carries the lens 54.

The microelectro-mechanical module 6 includes a support body 61 that surrounds the axis (X) of the hollow needle body 2 and that is disposed inside the needle passage 20 of the hollow needle body 2 from the needle opening 213 to the rearward end of the adapter 51, and a microelectro-mechanical component 62 that is disposed in the support body 61 proximally of the needle opening 213 to detect physiological characteristics of the body cavity. The support body 61 has a mounting hole 611, and a channel hole 612 that is formed in the support body for guiding a fluid flow. The mounting hole 611 and the channel hole 612 penetrate through the support body 61 in the direction parallel to the axis (X) of the outer tubular wall 21 and extend to the needle opening 213. In clinical implementation, the mounting hole 611 may receive the microelectro-mechanical component 62 and permit passage of electrical cables (not shown).

While the light emitter 43 is disposed inside the adapter 51 in the embodiment, it may also be disposed outside the adapter 51 in other embodiments of the present disclosure. On the other hand, the image sensor 52 and the microelectro-mechanical component 6 may be connected electrically to a control device (not shown) such that the control device is able to verify information received from the image sensor 52 and the microelectro-mechanical component 6.

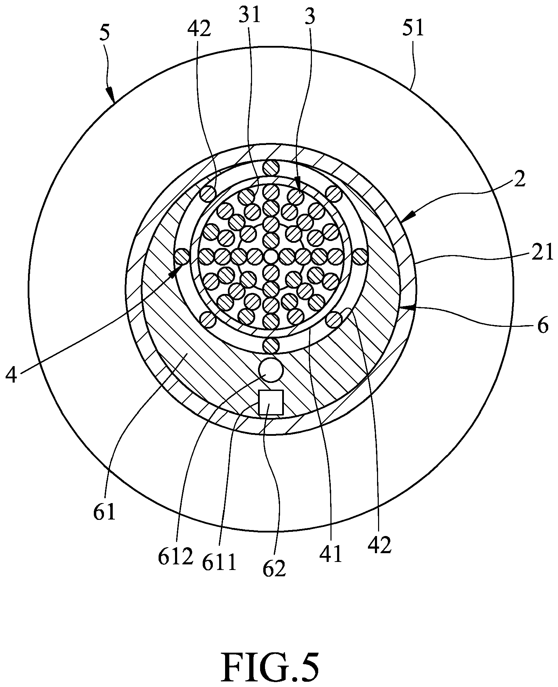

Referring to Figures 4 and 5, by virtue of the forward penetrating end 211 and the penetrating section 212 of the hollow needle body 2, the medical probe may penetrate into the body cavity of a patient during the penetrating process of a surgery. Meanwhile, the light emitter 43 emits the light to propagate from the light entrance ends 422 of the light guide rods 42 to the light exit ends 421 of the light guide rods 42 such that the light is delivered through the needle opening 213 to the body cavity in front of the forward penetrating end 211 of the hollow needle body 2.

Since the light entering region 311 of the optical fibers 31 is small and face forwardly from the needle opening 213 of the hollow needle body 2, the light reflected from the body cavity to the needle body 2 may enter the light entering region 311 and propagate through the optical fibers 31 inside the needle passage 20, and may be focused by the lens 54 for detection by the image sensor 52 so as to obtain image in front of the hollow needle body 2. If an abnormal condition (e.g., tissue adhesion) is observed during the penetrating process of the surgery, the location to be penetrated may be changed to avoid high-risk area.

It is worth mentioning that a syringe (not shown) may be connected to the channel hole 612 via the adapter 51 so as to inject liquors and/or testing liquid from the syringe to the body cavity through the channel hole 612, or to draw body fluid from the cavity to the syringe through the channel hole 612. In addition to that, the microelectro-mechanical component 62 disposed in the mounting hole 611 may detect physiological characteristics according to the body fluid and the tissue inside the cavity.

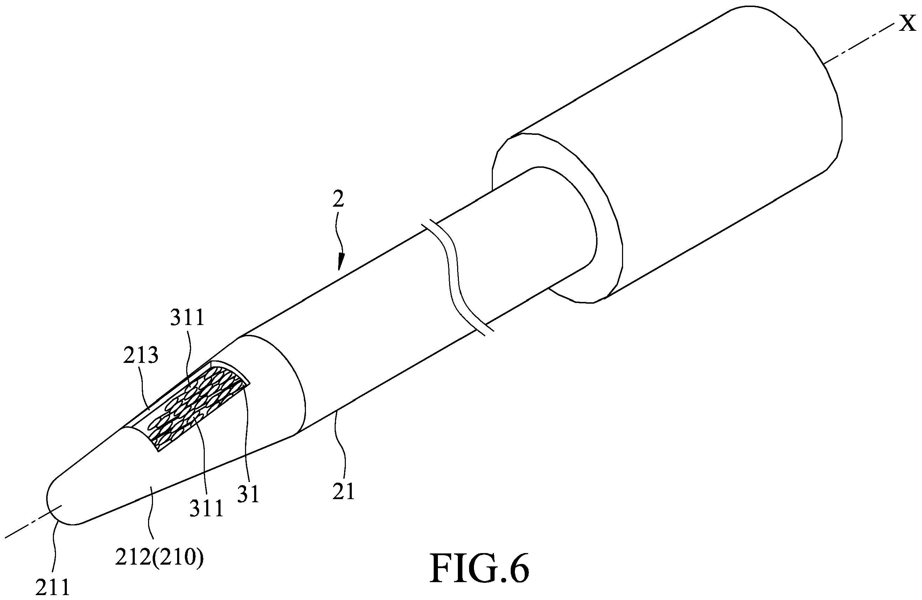

Referring to Figure 6, a second embodiment of the medical probe according to the present disclosure is shown to be similar to the first embodiment. The only difference resides in that the forward penetrating end 211 is blunt and situated on the axis (X) of the hollow needle body 2. The outer tubular wall 21 converges in the penetrating section 212 to form a converging wall section 210 that constitutes the penetrating section 212. The needle opening 213 is formed in one side of the converging wall section 210 to expose the light entering region 311 of the light-transmitting optical module 3.

Even though the blunt forward penetrating end 211 in this embodiment cannot directly penetrate skin and muscle of the patient, the medical probe of this embodiment may enter the body cavity of the patient after the skin and muscle is cut open by a surgical knife (e.g., a scalpel). In this embodiment, the effectiveness of the first embodiment may still be achieved.

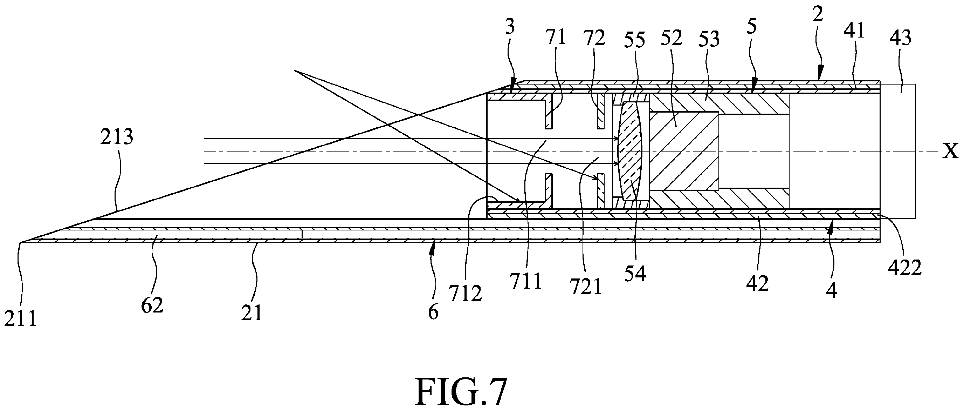

Referring to Figure 7, a third embodiment of the medical probe according to the present disclosure is shown, and is generally similar to the first embodiment. However, the needle passage 20 of the needle body 2 is in this embodiment is surrounded only by the outer tubular wall 21. The light emitter 43 is disposed at the rearward end 214 of the outer tubular wall 21, the light entrance ends 422 of the light guide rods 42 extend to the light emitter 43. The light-transmitting optical module 3 further includes first and second optical gratings 71, 72 that are disposed in the needle passage 20 in proximity to the needle opening 213. The first and second optical gratings 71, 72 have respectively first and second grating holes 711, 721 that are used as the light entering region 311. The axis (X) of the hollow needle body 2 extends through the first and second grating holes 711, 721. The first optical grating 71 in this embodiment further includes an inner surface 712 that surrounds the axis (X) of the hollow needle body 2 and that forms a light-unreflecting coating thereon.

The imaging module 5 in this embodiment is disposed in the needle passage 20 and is disposed within the inner tubular wall 41 of the needle body 2. The imaging module 5 includes an image sensor 52, a sensor carrier 53 carrying the image sensor 52, a lens 54 disposed between the second optical grating 72 and the image sensor 52, and a lens carrier 55 carrying the lens 54. The lens 54 is to focus the light reflected from the body cavity and passing through the first and second grating holes 711, 721.

The first and second grating holes 711, 721 only permit passage of the reflected light from the body cavity in front of the hollow needle body 2. Therefore, the reflected light coming from an upside of the hollow needle body 2 is excluded while the remaining reflected light passes through the first and second grating holes 711, 721. The reflected light passing through the first and second grating holes 711, 721 will propagate within the needle passage 20 and will be focused by the lens 54 so that an image in front of the hollow needle body 2 may be captured by the image sensor 52. In this embodiment, the effects of the first embodiment may still be achieved.

To conclude, the medical probe according to the present disclosure has the following advantages and effectiveness:
By virtue of the unique location designed for the light entering region 311 of the light-transmitting optical module 3, the imaging module 5 is able to capture the images of the body cavity in front of the hollow needle body 2 when the hollow needle body 2 penetrates the body cavity, thereby improving accuracy in determination of a proper surgical site for a surgery and reducing risks during the surgery.

## Claims

1. A medical probe for examining a body cavity during surgery, the medical probe comprising:
a hollow needle body (2) defining a needle passage (20) extending along an axis (X) of said hollow needle body (2), and including an outer tubular wall (21) surrounding said needle passage (20), said outer tubular wall (21) including a penetrating section (212) that is formed with a forward penetrating end (211) and that has a cross-section smaller than a remainder of said outer tubular wall (21), and a needle opening (213) formed in said penetrating section (212) and communicated with said needle passage (20);
a light-transmitting optical module (3) disposed inside said needle passage (20), and including a light entering region (311) that extends to and is exposed from said needle opening (213) to receive light from the body cavity, said light-transmitting optical module (3) transmitting the light within said needle passage (20); and
an imaging module (5) disposed in said needle passage (20) to capture an image carried by the light transmitted through said light-transmitting optical module (3); and being **characterized by** a microelectro-mechanical module (6), which includes a support body (61) disposed inside said needle passage (20), and a microelectro-mechanical component (62) disposed in said support body (61) proximally of said needle opening (213) to detect physiological characteristics of the body cavity.

2. The medical probe of Claim 1, **characterized in that** said outer tubular wall (21) is beveled to form said penetrating section (212) so that said penetrating section (212) has a beveled annular surface (215) that is inclined with respect to the axis (X) of said hollow needle body (2) and that confines said needle opening (213), said forward penetrating end (211) being situated at one side of said beveled annular surface offset from the axis (X).

3. The medical probe of Claim 1, **characterized in that** said forward penetrating end (211) is blunt and is situated on the axis (X) of said hollow needle body (2), said outer tubular wall (21) converging in said penetrating section (212) to form a converging wall section (210) that constitutes said penetrating section (212), said needle opening (213) being formed in one side of said converging wall section (210).

4. The medical probe of any one of Claims 1 to 3, **characterized in that** said support body (61) has a channel hole (612) formed therein for guiding a fluid flow.

5. The medical probe of Claim 4, **characterized in that** said channel hole (612) penetrates through said support body (61) in a direction parallel to the axis (X) of said outer tubular wall (21), said support body (61) further having a mounting hole (611) to receive said microelectro-mechanical component (62).

6. The medical probe of any one of Claims 1 to 5, **characterized in that** said light-transmitting optical module (3) further includes a plurality of optical fibers (31) that are disposed inside said needle passage (213) along the axis (X) and that extend from said needle opening (213) to said imaging module (5), said optical fibers (31) being beveled to respectively have beveled surfaces that cooperatively form said light entering region (311) of said light-transmitting optical module (3) in said needle opening (213).

7. The medical probe of any one of Claims 1 to 6, **characterized in that** said light-transmitting optical module (3) further includes an optical grating (71, 72) that is disposed in said needle passage (20) in proximity to said needle opening (213) and that has a grating hole (711, 721) to be used as said light entering region (311), the axis (X) of said hollow needle body (2) extending through said grating hole (711, 721).

8. The medical probe of any one of Claims 1 to 7, further **characterized by** a light-emitting module (4) to emit light to pass through said needle passage (20) and to deliver the light to the body cavity through said needle opening (213).

9. The medical probe of Claim 8, **characterized in that** said hollow needle body (2) further includes an inner tubular wall (41) disposed within said outer tubular wall (21) and surrounding said light-transmitting optical module (3), said light-emitting module (4) including a plurality of light guide rods (42) extending along the axis (X) of said hollow needle body (2) and disposed around said inner tubular wall (41), and a light emitter (43) distal from said needle opening (213), each of said light guide rods (42) having a light entrance end (422) proximal to said light emitter (43) to receive light from said light emitter (43), and a light exit end (421) extending to and exposed from said needle opening (213) to deliver the light through said needle opening (213) to the body cavity.

10. The medical probe of Claim 9, **characterized in that** said imaging module (5) includes an image sensor (52), a sensor carrier (53) carrying said image sensor (52) and inserted into said inner tubular wall (41), a lens (54) disposed between said light-transmitting optical module (3) and said image sensor (52) to focus the light, and a lens carrier (55) carrying said lens (54) and inserted into said inner tubular wall (41).

11. The medical probe of any one of Claims 9 and 10, **characterized in that** said hollow needle body (2) further includes an adapter (51) connected to a rearward end (214) of said outer tubular wall (21) oppositely of said forward penetrating end (212), said imaging module (5) including an image sensor (52) disposed inside said adapter (51), a sensor carrier (53) inserted in said adapter (51) and carrying said image sensor (52), a lens (54) disposed between said light-transmitting optical module (3) and said image sensor (52) to focus light, and a lens carrier (55) inserted into said adapter (51) and carrying said lens (54).

## Patentansprüche

1. Medizinische Sonde zur Untersuchung einer Körperhöhle während einem chirurgischen Eingriff, wobei die medizinische Sonde folgendes umfasst:
einen Hohlnadelkörper (2), der einen Nadeldurchgang (20) definiert, der sich entlang einer Achse (X) des genannten Hohlnadelkörpers (2) erstreckt und eine äußere röhrenförmige Wand (21) aufweist, die den genannten Nadeldurchgang (20) umgibt, wobei die genannte äußere röhrenförmige Wand (21) einen penetrierenden Abschnitt (212) aufweist, der mit einem vorderen penetrierenden Ende (211) ausgebildet ist und einen Querschnitt aufweist, der kleiner ist als ein Rest der genannten äußeren röhrenförmigen Wand (21), und wobei eine Nadelöffnung (213) in dem genannten penetrierenden Abschnitt (212) ausgebildet ist und in Kommunikation mit dem genannten Nadeldurchgang steht;
ein Licht übertragendes optisches Modul (3), das sich in dem genannten Nadeldurchgang (20) befindet und einen Lichteintrittsbereich (20) aufweist, der sich zu der genannten Nadelöffnung (213) erstreckt und von dieser exponiert ist, so dass er Licht von der Körperhöhle empfängt, wobei das genannte Licht übertragende optische Modul (3) das Licht in dem genannten Nadeldurchgang (20) überträgt; und
ein Bilddarstellungsmodul (5), das sich in dem genannten Nadeldurchgang (20) befindet, um ein Bild zu erfassen, das durch das Licht mitgeführt wird, das durch das genannte Licht übertragende optische Modul übertragen wird; und **gekennzeichnet durch**:
ein mikroelektromechanisches Modul (6), das einen Stützkörper (61) aufweist, der sich in dem genannten Nadeldurchgang (20) befindet, und eine mikroelektromechanische Komponente (62), die sich in dem genannten Stützkörper (61) proximal zu der genannten Nadelöffnung (213) befindet, um physiologische Eigenschaften der genannten Körperhöhle zu erkennen.

2. Medizinische Sonde nach Anspruch 1, **dadurch gekennzeichnet, dass** die genannte äußere röhrenförmige Wand (21) so abgeschrägt ist, dass sie den genannten penetrierenden Abschnitt (212) bildet, so dass der genannte penetrierende Abschnitt (212) eine abgeschrägte ringförmige Oberfläche (215) aufweist, die im Verhältnis zu der Achse (X) des genannten Hohlnadelkörpers (2) geneigt ist, und welche die genannte Nadelöffnung (213) begrenzt, wobei sich das genannte vordere penetrierende Ende (211) auf einer Seite der genannten abgeschrägten ringförmigen Oberfläche, versetzt von der Achse (X) angeordnet befindet.

3. Medizinische Sonde nach Anspruch 1, **dadurch gekennzeichnet, dass** das genannte vordere penetrierende Ende (211) stumpf ist und sich an der Achse (X) des genannten Hohlnadelkörpers (2) befindet, wobei die genannte äußere röhrenförmige Wand (21) in dem genannten penetrierenden Abschnitt (212) konvergiert, so dass ein konvergierender Wandabschnitt (210) gebildet wird, der den genannten penetrierenden Abschnitt (212) bildet, wobei die genannte Nadelöffnung (213) in einer Seite des genannten konvergierenden Wandabschnitts (210) ausgebildet ist.

4. Medizinische Sonde nach einem der Ansprüche 1 bis 3, **dadurch gekennzeichnet, dass** der genannte Stützkörper (61) eine darin ausgebildete Kanalöffnung (612) aufweist, um einen Fluidfluss zu leiten.

5. Medizinische Sonde nach Anspruch 4, **dadurch gekennzeichnet, dass** die genannte Kanalöffnung (612) in eine Richtung, die parallel ist zu der Achse (X) der genannten äußeren röhrenförmigen Wand (21), durch den genannten Stützkörper (61) penetriert, wobei der genannte Stützkörper (61) ferner eine Befestigungsöffnung (611) zur Aufnahme der genannten mikroelektromechanischen Komponente (62) aufweist.

6. Medizinische Sonde nach einem der Ansprüche 1 bis 5, **dadurch gekennzeichnet, dass** das genannte Licht übertragende optische Modul (3) ferner eine Mehrzahl von Lichtwellenleitern (31) aufweist, die entlang der Achse (X) in dem genannten Nadeldurchgang (213) angeordnet sind und die sich von der genannten Nadelöffnung (213) zu dem genannten Bilddarstellungsmodul (5) erstrecken, wobei die genannten Lichtwellenleiter (31) abgeschrägt sind, so dass sie jeweils abgeschrägte Oberflächen aufweisen, die zusammenwirkend den genannten Lichteintrittsbereich (311) des genannten Licht übertragenden optischen Moduls (3) in der genannten Nadelöffnung (213) bilden.

7. Medizinische Sonde nach einem der Ansprüche 1 bis 6, **dadurch gekennzeichnet, dass** das genannte Licht übertragende optische Modul (3) ferner ein optisches Gitter (71, 72) aufweist, das sich in dem genannten Nadeldurchgang (20) proximal zu der genannten Nadelöffnung (213) befindet, und das eine Gitteröffnung (711, 721) zur Verwendung als Lichteintrittsbereich (311) aufweist, wobei sich die Achse (X) des genannten Hohlnadelkörpers (2) durch die genannte Gitteröffnung (711, 721) erstreckt.

8. Medizinische Sonde nach einem der Ansprüche 1 bis 7, ferner **gekennzeichnet durch** ein lichtemittierendes Modul (4) zur Emission von Licht, so dass dieses durch den genannten Nadeldurchgang (20) tritt und so dass das Licht durch die genannte Nadelöffnung (213) der Körperhöhle zugeführt wird.

9. Medizinische Sonde nach Anspruch 8, **dadurch gekennzeichnet, dass** der genannte Hohlnadelkörper (2) ferner eine innere röhrenförmige Wand (41) aufweist, die sich in der genannten äußeren röhrenförmigen Wand (21) befindet und das genannte Licht übertragende optische Modul (3) umgibt, wobei das genannte lichtemittierende Modul (4) eine Mehrzahl von Lichtleiterstäben (42) aufweist, die sich entlang der Achse (X) des genannten Hohlnadelkörpers (2) erstrecken und um die genannte innere röhrenförmige Wand (41) angeordnet sind, und mit einem Lichtsender (43), der distal zu der genannten Nadelöffnung (213) angeordnet ist, wobei jeder der genannten Lichtleiterstäbe (42) ein Lichteintrittsende (422) proximal zu dem genannten Lichtsender (42) aufweist, um Licht von dem genannten Lichtsender (43) zu empfangen, und mit einem Lichtaustrittsende (421), das sich von der genannten Nadelöffnung (213) erstreckt und von dieser exponiert ist, um das Licht durch die genannte Nadelöffnung (213) der Körperhöhle zuzuführen.

10. Medizinische Sonde nach Anspruch 9, **dadurch gekennzeichnet, dass** das genannte Bilddarstellungsmodul (5) folgendes aufweist: einen Bildsensor (52), einen Sensorträger (53), der den genannten Bildsensor (52) trägt und in die genannte innere röhrenförmige Wand (41) eingeführt ist, eine Linse (54), die sich zwischen dem genannten Licht übertragenden optischen Modul (3) und dem genannten Bildsensor (52) befindet, um das Licht zu fokussieren, und einen Linsenträger (55), der die genannte Linse (54) trägt und in die genannte innere röhrenförmige Wand (41) eingeführt ist.

11. Medizinische Sonde nach Anspruch 9 oder 10, **dadurch gekennzeichnet, dass** der genannte Hohlnadelkörper (2) ferner einen Adapter (51) aufweist, der mit einem hinteren Ende (214) der genannten äußeren röhrenförmigen Wand (21) entgegengesetzt zu dem genannten vorderen penetrierenden Ende (212) verbunden ist, wobei das genannte Bilddarstellungsmodul (5) folgendes aufweist: einen Bildsensor (52), der sich in dem genannten Adapter (51) befindet, einen Sensorträger (53), der in den genannten Adapter (51) eingeführt ist und den genannten Bildsensor (52) trägt, eine Linse (54), die sich zwischen dem genannten Licht übertragenden optischen Modul (3) und dem genannten Bildsensor (52) befindet, um das Licht zu fokussieren, und einen Linsenträger (55), der in den genannten Adapter (51) eingeführt ist und die genannte Linse (54) trägt.

## Revendications

1. Sonde médicale pour examiner une cavité corporelle au cours d'une opération chirurgicale, la sonde médicale comprenant :
un corps d'aiguille creux (2) définissant un passage d'aiguille (20) s'étendant le long d'un axe (X) dudit corps d'aiguille creux (2), et comprenant une paroi tubulaire externe (21) entourant ledit passage d'aiguille (20), ladite paroi tubulaire externe (21) comprenant une section pénétrante (212) qui est formée avec une extrémité pénétrante avant (211) et a une section transversale plus petite qu'un reste de ladite paroi tubulaire externe (21), et une ouverture d'aiguille (213) formée dans ladite section pénétrante (212) et en communication avec ledit passage d'aiguille (20) ;
un module optique transmetteur de lumière (3) disposé à l'intérieur dudit passage d'aiguille (20), et comprenant une lumière d'entrée de lumière (311) qui s'étend jusqu'à et est exposée à partir de ladite ouverture d'aiguille (213) pour recevoir de la lumière provenant de la cavité corporelle, ledit module optique transmetteur de lumière (3) transmettant la lumière dans ledit passage d'aiguille (20) ; et
un module d'imagerie (5) disposé dans ledit passage d'aiguille (20) pour capturer une image véhiculée par la lumière transmise à travers ledit module optique transmetteur de lumière (3) ;
et **caractérisée par** un module microélectro-mécanique (6), qui comprend un corps de support (61) disposé à l'intérieur dudit passage d'aiguille (20), et un composant microélectro-mécanique (62) disposé dans ledit corps de support (61) à proximité de ladite ouverture d'aiguille (213) pour détecter les caractéristiques physiologiques de la cavité corporelle.

2. Sonde médicale selon la revendication 1, **caractérisée en ce que** ladite paroi tubulaire externe (21) est biseautée pour former ladite section pénétrante (212) de sorte que ladite section pénétrante (212) ait une surface annulaire biseautée (215) qui est inclinée par rapport à l'axe (X) dudit corps d'aiguille creux (2) et qui confine ladite ouverture d'aiguille (213), ladite extrémité pénétrante vers l'avant (211) étant située sur un côté de ladite surface annulaire biseautée décalé dudit axe (X).

3. Sonde médicale selon la revendication 1, **caractérisée en ce que** ladite extrémité pénétrante vers l'avant (211) est émoussée et est située sur l'axe (X) dudit corps d'aiguille creux (2), ladite paroi tubulaire externe (21) convergeant dans ladite section pénétrante (212) pour former une section de paroi convergente (210) qui constitue ladite section pénétrante (212), ladite ouverture d'aiguille (213) étant formée dans un côté de ladite section de paroi convergente (210).

4. Sonde médicale selon l'une quelconque des revendications 1 à 3, **caractérisée en ce que** ledit corps de support (61) a un trou de canal (612) formé en son sein pour guider un flux de fluide.

5. Sonde médicale selon la revendication 4, **caractérisée en ce que** ledit trou de canal (612) pénètre à travers ledit corps de support (61) dans une direction parallèle à l'axe (X) de ladite paroi tubulaire externe (21), ledit support de corps (61) ayant en outre un trou de fixation (611) pour recevoir ledit composant microélectro-mécanique (62).

6. Sonde médicale selon l'une quelconque des revendications 1 à 5, **caractérisée en ce que** ledit module optique transmetteur de lumière (3) comprend en outre une pluralité de fibres optiques (31) qui sont disposées à l'intérieur dudit passage d'aiguille (213) le long de l'axe (X) et qui s'étendent de ladite ouverture d'aiguille (213) vers ledit module d'imagerie (5), lesdites fibres optiques (31) étant biseautées pour respectivement avoir des surfaces biseautées qui conjointement forment ladite région d'entrée de lumière (311) dudit module optique transmetteur de lumière (3) dans ladite ouverture d'aiguille (213).

7. Sonde médicale selon l'une quelconque des revendications 1 à 6, **caractérisée en ce que** ledit module optique transmetteur de lumière (3) comprend en outre un réseau optique (71, 72) qui est disposé dans ledit passage d'aiguille (20) à proximité de ladite ouverture d'aiguille (213) et qui a un trou de réseau (711, 721) à utiliser comme ladite région d'entrée de lumière (311), l'axe (X) dudit corps d'aiguille creux (2) s'étendant à travers ledit trou de réseau (711, 721).

8. Sonde médicale selon l'une quelconque des revendications 1 à 7, **caractérisée en outre par** un module électroluminescent (4) pour émettre de la lumière destinée à passer à travers ledit passage d'aiguille (20) et amener la lumière à la cavité du corps par l'intermédiaire de ladite ouverture d'aiguille (213).

9. Sonde médicale selon la revendication 8, **caractérisée en ce que** ledit corps d'aiguille creux (2) comprend en outre une paroi tubulaire interne (41) disposée dans ladite paroi tubulaire externe (21) et entourant ledit module optique transmetteur de lumière (3), ledit module électroluminescent (4) comprenant une pluralité de tiges de guidage de lumière (42) s'étendant le long de l'axe (X) dudit corps d'aiguille creux (2) et disposées autour de ladite paroi tubulaire interne (41), et un émetteur lumineux (43) distal de ladite ouverture d'aiguille (213), chacune desdites tiges de guidage de lumière (42) ayant une extrémité d'entrée de lumière (422) proximale audit émetteur électroluminescent (43) pour recevoir de la lumière dudit émetteur électroluminescent (43), et une extrémité de sortie de lumière (421) s'étendant jusqu'à et exposée à partir de ladite ouverture d'aiguille (213) pour amener la lumière à travers ladite ouverture d'aiguille (213) à la cavité de corps.

10. Sonde médicale selon la revendication 9, **caractérisée en ce que** ledit module d'imagerie (5) comprend un capteur d'image (52), un support de capteur (53) portant ledit capteur d'image (52) et inséré dans ladite paroi tubulaire interne (41), une lentille (54) disposée entre ledit module optique transmetteur de lumière (3) et ledit capteur d'image (52) pour concentrer la lumière, et un support de lentille (55) portant ladite lentille (54) et inséré dans ladite paroi tubulaire interne (41).

11. Sonde médicale selon l'une quelconque des revendications 9 et 10, **caractérisée en ce que** ledit corps d'aiguille creux (2) comprend en outre un adaptateur (51) connecté à une extrémité arrière (214) de ladite paroi tubulaire externe (21) à l'opposé de ladite extrémité pénétrante avant (212), ledit module d'imagerie (5) comprenant un capteur d'image (52) disposé à l'intérieur dudit adaptateur (51), un support de capteur (53) inséré dans ledit adaptateur (51) et portant ledit capteur d'image (52), une lentille (54) disposée entre ledit module optique transmetteur de lumière (3) et ledit capteur d'image (52) pour concentrer la lumière, et un support de lentille (55) inséré dans ledit adaptateur (51) et portant ladite lentille (54).
